# EUROPEAN PATENT SPECIFICATION

(11) **EP 1 275 383 B1**
(45) Date of publication and mention of the grant of the patent: **31.03.2004**
(21) Application number: 02012184.4
(22) Date of filing: 03.06.2002
(51) Int. Cl.: A61K 31/135, A61K 31/137, A61K 9/20, A61K 9/22

(54) **Modified release pharmaceutical composition containing Bupropion HCI as active substance**
Formulierung mit gesteuerter Wirkstoffabgabe enthaltend Bupropion-HCI als Wirkstoff
Composition pharmaceutique à libération contrôlée contenant du bupropion-HCl en tant qu'agent actif

(30) Priority: 09.07.2001 IT MI20011457
(43) Date of publication of application: 15.01.2003
(73) Proprietor: Valpharma S.A., 47899 Serravalle (SM)
(72) Inventor: Valducci, Roberto, 47039 Savignano Sul Rubicone (FC) (IT); Alighieri, Tiziano, 47900 Rimini (IT); Avanessian, Serozh, 47900 Rimini (IT)
(74) Representative: Negrini, Elena

(56) References cited:
- WO-A-94/04138
- US-A- 4 393 078
- US-A- 6 096 341
- US-B1- 6 194 002
- US-B1- 6 238 697

## Description

### STATE OF THE ART

Bupropion HCI is a substance having interesting pharmacological characteristics, similar to those of the tricyclic antidepressants.

Nevertheless Bupropion HCI has an elevated hygroscopicity and susceptibility to the decomposition.

For this reason various compositions with stabilizing intent have been studied.

For example, the patents n° WO 95/03781 and USP 5,541,231 describe compositions in solid form in which the presence of various acid substances gives stability to Bupropion HCl.

The patents n° USP 5,427,798 and EP 656775 describe controlled release tablets obtained through the technique of hydrophile matrixes based on hydroxypropylmethylcellulose.

However, the stability and the dissolution profile are not satisfactory.

Further modified release compositions are described in the patent n° EP 0171457, which provides for the preparation of a Bupropion HCI core with osmotic components, and then the coating of said core with a membrane insoluble in water but permeable. In such membrane, soluble water substances are suspended, whose solubilization allows to "perforate" the membrane thus enabling the release of Bupropion HCl.

These techniques have the disadvantage of being very complicated and laborious.

### SUMMARY

Now we have found pharmaceutical compositions containing Bupropion HCl as active substance, which allow to overcome the disadvantages of the prior art.

Such compositions are in tablet form and are characterized by the simultaneous presence of hydrophilic substances and hydrophobic substances.

In particular, the compositions according to this invention include hydroxypropylmethylcellulose, or polyethylene oxide, stearic acid and carnauba wax and an excipient substance like lactose.

Using the various ingredients in the proper proportions it is possible to modulate the dissolution profile of Bupropion HCI as requested by the European Pharmacopoeia and to obtain tablets suitable for all the commonly utilized dosages.

Furthermore the compositions according to this invention show an elevated reproducibility of the chemical-physical characteristics and the dissolution profile.

### DESCRIPTION OF THE INVENTION

The characteristics and the advantages of the pharmaceutical compositions containing Bupropion HCI as active substance according to this invention will be better explained through the following detailed description and through the examples of preparation and characterization.

Such compositions are prepared in tablet form and include hydrophilic components and hydrophobic components.

A preferred preparation includes, besides Bupropion HCI (BP), hydroxypropylmethylcellulose (HPMC), stearic acid (SA) and carnauba wax (W), as well as an excipient substance like lactose (L). In another preferred preparation hydroxypropylmethylcellulose is replaced by polyethylene oxide.

For the tablet preparation, the various components in powder form are accurately mixed. The obtained mixture is extruded at a temperature ranging from 45°C to 65°C to obtain a granulate.

The granulate is mixed with lubricating substances commonly utilized in the pharmaceutical technique and then transformed into tablets.

Each tablet has a Bupropion HCI content ranging from 50 mg to 500 mg.

In the composition preparation according to this invention the various components are used in the following weight proportions:
- BP / (HPMC + SA + W) from 0,5 to 2;
- HPMC / SA / W from 1/1/1 to 1/4/4;
- BP / L from 1 to 2.

As stated above it comes out that the preparation process has the advantage of being realized through more simple operations in comparison with the prior art.

Furthermore it allows the obtainment of a stable, not hygroscopic, modified release composition, having reproducible characteristics and suitable for all the commonly used dosages.

For a better explanation of the invention the following examples are reported.

### EXAMPLE 1

In a Viani granulator, type ST 25, the following ingredients in powder form were mixed:

| | |
|---|---|
| Bupropion HCI | 9.000 g |
| Hydroxypropylmethylcellulose K 15 | 1.800 g |
| Lactose | 5.900 g |
| Stearic Acid | 1.800 g |
| Carnauba Wax | 1.800 g |

The obtained mixture was extruded through a Kahl extruder, model press 14-175, with a 0,8 mm net, maintaining the granulation temperature at 50°C. The so obtained granulate was mixed with lubricating substances (magnesium stearate and anhydrous colloidal silica in quantity of 2 and 1 mg per tablet, respectively) and, therefore, compressed into tablets. Each tablet having the average weight of 300 mg had a Bupropion HCI content of 150 mg. The tablets were characterized by the Bupropion HCI release utilizing the method of the European Pharmacopoeia (Paddle apparatus) and the following results were obtained:

| Percentage release | | | | |
|---|---|---|---|---|
| 1 h | 2 h | 4 h | 8 h | 12 h |
| 30 | 45 | 67 | 94 | 100 |

The above indicated tablets were coated and coloured to improve their appearance and protection: such coating _leaves the dissolution characteristics unchanged.

### EXAMPLE 2

With the same method described in example n. 1 a granulate with the following composition was prepared:

| | |
|---|---|
| Bupropion HCI | 590 g |
| Hydroxypropylmethylcellulose K100 | 96 g |
| Hydroxypropylmethylcellulose K4 | 100 g |
| Lactose | 648 g |
| Stearic Acid | 196 g |
| Carnauba Wax | 196 g |

With a portion of the granulate, tablets containing 100 mg of Bupropion HCl were prepared.

Utilizing the same method of the preceding example the following results could be obtained:

| Percentage release | | | | |
|---|---|---|---|---|
| 1 h | 2 h | 4 h | 8 h | 12 h |
| 34 | 51 | 72 | 96 | 103 |

With the remaining granulate, tablets containing 150 mg of active ingredient were prepared; the so obtained tablets have been coated and coloured. In the in vitro release evaluation the following results were obtained:

| Percentage release | | | | |
|---|---|---|---|---|
| 1 h | 2 h | 4 h | 8 h | 12 h |
| 30 | 43 | 62 | 85 | 101 |

### EXAMPLE 3

In a Z double jacket mixer granulator, warmed at 60°C, LLEAL model AM-5, granules with the following composition were prepared:

| | |
|---|---|
| Bupropion HCI | 300 g |
| Hydroxypropylmethylcellulose K100 | 100 g |
| Lactose | 200 g |
| Stearic Acid | 200 g |
| Carnauba Wax | 200 g |

The granules were forced through a net with 1000 micrometers aperture size. The so sieved granules were mixed with lubricants and transformed into tablets containing 100 mg of active ingredient: the tablets were analysed obtaining the following results:

| Percentage release | | | | |
|---|---|---|---|---|
| 1 h | 2 h | 4 h | 8 h | 12 h |
| 33 | 48 | 70 | 84 | 94 |

### EXAMPLE 4 (Comparison)

Example n. 3 was repeated, with the difference that in the preparation of the composition hydroxypropylmethylcellulose was not included and, therefore, the example was carried out without the hydrophilic component.

The tablets were analysed obtaining the following results:

| Percentage release | | | | | | | |
|---|---|---|---|---|---|---|---|
| 1 h | 2 h | 4 h | 6 h | 8 h | 12 h | 16 h | 20 h |
| 23,0 | 31,4 | 42,5 | 50,4 | 56,5 | 65,6 | 72,1 | 76,7 |

The active ingredient dissolution patterns turned out to be very slow and not capable of being modulated.

### EXAMPLE 5

Tablets with the same procedure and composition of example n. 3 were prepared, replacing Hydroxypropylmethylcellulose with Polyethylene Oxide having a molecular weight of 1.000.000; the analysed tablets gave the following results:

| Percentage release | | | | |
|---|---|---|---|---|
| 1 h | 2 h | 4 h | 8 h | |
| 39 | 62 | 84 | 100.5 | |

### EXAMPLE 6

In order to obtain a three layer tablet formulation the following granulates were prepared:
6.1 Bupropion HCI was mixed with the excipients in powder, wet with the PVP solution and forced in a net having 1000 micrometers aperture size:

| | |
|---|---|
| Bupropion HCI | 1.000 g |
| Lactose | 400 g |
| Microcrystalline Cellulose | 200 g |
| PVP at 20% in Ethanol | 320 g |

The granules were transferred into a desiccator and desiccated for 24 hours at 40°C.
6.2 Utilizing the method described in example n. 3, granules with the following composition were prepared:

| | |
|---|---|
| Bupropion HCI | 300 g |
| Hydroxypropylmethylcellulose K100 | 50 g |
| Lactose | 180 g |
| Stearic Acid | 180 g |
| Carnauba Wax | 180 g |

6.3 Utilizing the method described in example n. 3, granules with the following composition were prepared:

| | |
|---|---|
| Bupropion HCI | 300 g |
| Hydroxypropylmethylcellulose K100 | 80 g |
| Lactose | 150 g |
| Stearic Acid | 150 g |
| Carnauba Wax | 180 g |

The above described granulates were transformed into three layer tablets by tableting them in the following order:

| | |
|---|---|
| 1^{st} layer utilizing granulate 6.1 | 26% |
| 2^{nd} layer utilizing granulate 6.2 | 37% |
| 3^{rd} layer utilizing granulate 6.3 | 37% |

Each tablet had an active ingredient content of 100 mg.

The tablets were analysed after colouring obtaining the following dissolution profile:

| Percentage release | | | | |
|---|---|---|---|---|
| 1 h | 2 h | 4 h | 8 h | 12 h |
| 38 | 55.7 | 75.4 | 90 | 95.5 |

### EXAMPLE 7

Utilizing the granulates previously prepared in example n. 5, two layer tablets containing 150 mg of active ingredient each were prepared.

The two layers had the following composition:
- granulate 6.2 70%
- granulate 6.3 30%

The so obtained tablets were coloured and checked for the in vitro release obtaining the following results:

| Percentage release | | | | |
|---|---|---|---|---|
| 1 h | 2 h | 4h | 8 h | 12 h |
| 21 | 47 | 62 | 85 | 100 |

## Claims

1. Modified release pharmaceutical composition in tablet form containing Bupropion HCl as active substance, **characterized in that**, as components, hydroxypropylmethylcellulose, stearic acid and carnauba wax mixed with an excipient substance are included.

2. Composition according to claim 1, wherein such excipient substance is lactose.

3. Composition according to claim 1, wherein such components are present in a weight ratio ranging from 0,5 to 2 of Bupropion HCI and the sum of hydroxypropylmethylcellulose, stearic acid as well as camauba wax.

4. Composition according to claim 1, wherein the weight ratio "hydroxypropylmethylcellulose: stearic acid: carnauba wax" is ranging from "1:1:1" to "1:4:4".

5. Composition according to claim 2, wherein the weight ratio between Bupropion HCI and lactose is ranging from 1 and 2.

6. Composition according to claim 1, wherein the Bupropion HCI content for each tablet is ranging from 50 mg to 500 mg.

## Patentansprüche

1. Pharmazeutische Formulierung mit gesteuerter Wirkstoffabgabe in Tablettenform enthaltend Bupropion-HCl als Wirkstoff, **dadurch gekennzeichnet, dass** als Bestandteile Hydroxipropylmethylcellulose, Stearinsäure und Karnaubawachs gemischt mit einem Bindemittel enthalten sind.

2. Formulierung nach Anspruch 1, bei welcher das Bindemittel Laktose ist.

3. Formulierung nach Anspruch 1, bei welcher die Bestandteile in einem Gewichtsverhältnis im Bereich von 0,5 bis 2 von Bupropion-HCl und der Summe aus Hydroxipropylmethylcellulose, Stearinsäure und Karnaubawachs vorliegen.

4. Formulierung nach Anspruch 1, bei welcher das Gewichtsverhältnis "Hydroxipropylmethylcellulose : Stearinsäure : Karnaubawachs" im Bereich von "1:1:1" bis "1:4:4" liegt.

5. Formulierung nach Anspruch 2, bei welcher das Gewichtsverhältnis zwischen Bupropion-HCl und Laktose im Bereich von 1 bis 2 liegt.

6. Formulierung nach Anspruch 1, bei welcher der Bupropion-HCl-Gehalt für jede Tablette im Bereich von 50 mg bis 500 mg liegt.

## Revendications

1. Composition pharmaceutique à libération contrôlée, sous forme de comprimé, contenant du Bupropion HCl comme principe actif, **caractérisée en ce que** sont incorporés, à titre de constituants, de l'hydroxypropylméthylcellulose, de l'acide stéarique et de la cire de carnauba mélangés avec une substance excipient.

2. Composition selon la revendication 1, dans laquelle cette substance excipient est le lactose.

3. Composition selon la revendication 1, dans laquelle ces constituants sont présents dans un rapport pondéral entre le Bupropion HCl et la somme des constituants hydroxypropylméthylcellulose, acide stéarique ainsi que cire de carnauba s'échelonnant de 0,5 à 2.

4. Composition selon la revendication 1, dans laquelle le rapport pondéral "hydroxypropylméthylcellulose/acide stéarique/cire de carnauba" s'échelonne de "1/1/1" à "1/4/4".

5. Composition selon la revendication 2, dans laquelle le rapport pondéral entre le Bupropion HCl et le lactose s'échelonne de 1 à 2.

6. Composition selon la revendication 1, dans laquelle la teneur en Bupropion HCl de chaque comprimé s'échelonne de 50 mg à 500 mg.
